# EUROPÄISCHE PATENTANMELDUNG

(11) **EP 1 544 305 A1**
(43) Veröffentlichungstag der Anmeldung: **22.06.2005**
(21) Anmeldenummer: 03029139.7
(22) Anmeldetag: 18.12.2003
(51) Int. Cl.: C12N 15/86, C12N 15/62, A61K 47/48

(54) **Adapter zum Ankoppeln einer an einer Zelloberfläche anzukoppelnden Substanz**

(71) Anmelder: Medizinische Hochschule Hannover, 30625 Hannover (DE)
(72) Erfinder: Kubicka, Stefan, 30519 Hannover (DE); Kühnel, Florian, 30613 Hannover (DE); Schulte, Bernd, 49809 Lingen (DE)
(74) Vertreter: Eisenführ, Speiser & Partner

(57) **Zusammenfassung**

Die Erfindung betrifft Adapter zum Ankoppeln einer an einer Zelloberfläche anzukoppelnden Substanz. Insbesondere betrifft die Erfindung Adapter zum Ankoppeln eines adenoviralen Fiberknob-Proteins an eine Zelloberfläche, sowie für diese Adapter codierende Nucleinsäuren, Viren und Verfahren zu ihrer Verwendung. Die Erfindung betrifft auch Substanzen und Verfahren zum Vermitteln und/oder Verbessern des Ankoppelns von Substanzen wie Adenovirus-Partikel an eine Zelloberfläche, die wenig oder gar keinen Coxsackie Adenovirus-Rezeptor enthält.

SEQUENCE LISTING

<110>  Medizinische Hochschule Hannover

<120>  Adapter zum Ankoppeln einer an einer Zelloberfläche anzukoppelnden
Substanz

<130>  MA 7394-01DE

<160>  11

<170>  PatentIn version 3.1

<210>  1

<211>  33

<212>  DNA

<213>  Artificial Sequence

<220>

<223>  human Coxsackie Adenovirus Receptor (hCAR) 5'-Primer

<220>

<221>  misc_recomb

<222>  (3)..(8)

<223>  KpnI restriction site

<220>

<221>  misc_feature

<222>  (9)..(11)

<223>  translation start codon of hCAR

<400>  1
gtggtaccat ggcgctcctg ctgtgcttcg tgc                                    33

<210>  2

<211>  42

<212>  DNA

<213>  Artificial Sequence

<220>

<223>  human Coxsackie Adenovirus Receptor (hCAR) 3'-Primer

<220>

<221>  misc_recomb

<222>  (3)..(10)

<223>  NotI restriction site

<220>

<221>  misc_feature

<222>  (11)..(42)

<223>  complementary bases, corresponding to bases 776 to 807 of hCAR (a
       ccession no. NM 001338)

<400>  2
tagcggccgc ctttatttga aggagggaca acgtttagac gc                          42

<210> 3

<211> 75

<212> DNA

<213> Artificial Sequence

<220>

<223> 5'-PCR primer for HIV tat basic region

<220>

<221> misc_feature

<222> (36)..(65)

<223> "sense" oligonucleotide, corresponding to bases nos. 5518-5547 of
 the HIV genome (accession no. NC 001802)

<220>

<221> misc_recomb

<222> (4)..(11)

<223> NotI restriction site

<220>

<221> misc_recomb

<222> (68)..(73)

<223> XbaI restriction site

<400> 3
aaagcggccg cggaggagga agtggaggag gaggaggcag gaagaagcgg agacagcgac 60

gaagaggtct agaaa 75

<210>  4

<211>  10

<212>  PRT

<213>  Artificial Sequence

<220>

<223>  basic region of HIV tat protein

<400>  4

Gly Arg Lys Lys Arg Arg Gln Arg Arg Arg
1               5                   10

<210>  5

<211>  75

<212>  DNA

<213>  Artificial Sequence

<220>

<223>  3'-PCR primer for HIV tat basic region

<220>

<221>  misc_feature

<222>  (1)..(75)

<223>  complementary to pCAR(ex)TAT48-57 5'

<400>  5
tttctagacc tcttcgtcgc tgtctccgct tcttcctgcc tcctcctcct ccacttcctc      60

ctccgcggcc gcttt                                                        75

<210>  6

<211>  72

<212>  DNA

<213>  Artificial Sequence

<220>

<223>  5'-PCR primer for 9 Arg peptide

<220>

<221>  misc_recomb

<222>  (4)..(11)

<223>  NotI restriction site

<220>

<221>  misc_recomb

<222>  (65)..(70)

<223>  XbaI restriction site

<220>

<221>  CDS

<222>  (36)..(62)

<223>  oding for 9 Arg ("RRRRRRRRR")

<400>  6
aaagcggccg cggaggagga agtggaggag gagga cgt cgc cga cgg aga agg        53
                                       Arg Arg Arg Arg Arg Arg
                                       1               5

aga cgt aga ggtctagaaa                                                72
Arg Arg Arg

<210> 7

<211> 9

<212> PRT

<213> Artificial Sequence

<220>

<223> 5'-PCR primer for 9 Arg peptide

<400> 7

Arg Arg Arg Arg Arg Arg Arg Arg Arg
1               5

<210> 8

<211> 72

<212> DNA

<213> Artificial Sequence

<220>

<223> 3'-PCR primer for 9 Arg peptide

<220>

<221> misc_feature

<222> (1)..(72)

<223> complementary to pCAR(ex)-9xArg 5'

<400> 8
tttctagacc tctacgtctc cttctccgtc ggcgacgtcc tcctcctcca cttcctcctc     60

cgcggccgct tt                                                         72

<210> 9

<211> 80

<212> DNA

<213> Artificial Sequence

<220>

<223> 5'-PCR primer for antennapedia homeobox-protein fragment

<220>

<221> misc_recomb

<222> (3)..(10)

<223> NotI restriction site

<220>

<221> misc_recomb

<222> (73)..(78)

<223> XbaI restriction site

<220>

<221> misc_feature

<222> (23)..(70)

<223> partial coding sequence of Euprymna scolopes antennapedia homeodo
main protein (accession no.: AY052758), coding for amino acids 62
-77

<400> 9
aagcggccgc ggaggaggag gaagacagat caaaatatgg ttccaaaacc ggcgcatgaa 60

atggaagaaa ggtctagaaa 80

<210> 10

<211> 16

<212> PRT

<213> Artificial Sequence

<220>

<223> antennapedia homeobox protein fragment from Euprymna scolopes

<400> 10

Arg Gln Ile Lys Ile Trp Phe Gln Asn Arg Arg Met Lys Trp Lys Lys
1               5                   10                  15

<210> 11

<211> 80

<212> DNA

<213> Artificial Sequence

<220>

<223> 3'-PCR primer for antennapedia homeobox-protein fragment

<220>

<221> misc_feature

<222> (1)..(80)

<223> complementary to pCAR(ex)-AntP62-77 5'

<400> 11
tttctagacc tttcttccat ttcatgcgcc ggttttggaa ccatattttg atctgtcttc 60

ctcctcctcc gcggccgctt 80

## Beschreibung

Die Erfindung betrifft Adapter zum Ankoppeln einer an einer Zelloberfläche anzukoppelnden Substanz. Insbesondere betrifft die Erfindung Adapter zum Ankoppeln eines adenoviralen Fiberknob-Proteins an eine Zelloberfläche, sowie für diese Adapter codierende Nucleinsäuren, Viren und Verfahren zu ihrer Verwendung. Die Erfindung betrifft auch Substanzen und Verfahren zum Vermitteln und/oder Verbessern des Ankoppelns von Substanzen wie Adenovirus-Partikel an eine Zelloberfläche, die wenig oder gar keinen Coxsackie Adenovirus-Rezeptor enthält.

Zum Transfer heterologer Nucleinsäuren in eukaryontische Zellen, insbesondere Säugerzellen, werden häufig transgene Adenoviren verwendet. Diese tragen auf der Oberfläche des Virus-Partikels ein Fiberknob genanntes Protein, dass von einem auf der Oberfläche der zu behandelnden Zelle (Zielzelle) angeordneten Coxsackie Adenovirus Rezeptor (CAR; Bergelson, J.M. et al., Isolation of a common receptor for Coxsackie B viruses and adenovirusses 2 and 5; Science 275 (1997), 1320-1323) genannten Rezeptorprotein erkannt und gebunden werden kann. Die Bindung des Fiberknob-Proteins an CAR gilt als geschwindigkeitsbestimmender Schritt des adenoviralen Infektionsprozesses. Es wird angenommen, dass nach dem Binden des Coxsackie Adenovirus Rezeptors an das Fiberknob-Protein und dem damit einhergehenden Ankoppeln des Virus-Partikels an der Zelloberfläche ein weiteres Adenovirus-Hüllprotein, das Pentonprotein, an einen Bestandteil der Zelloberfläche bindet, nämlich an eines oder mehrere Integrine, wodurch eine Endocytose des Virus-Partikels in die Zielzelle ausgelöst wird.

Die Abhängigkeit von Adenoviren von dem Vorhandensein des Coxsackie Adenovirus Rezeptors auf der Oberfläche der Zielzelle schränkt die Verwendbarkeit von Adenoviren zum Transfer heterologer Nucleinsäuren in eukaryontische Zellen ein (Kim, M., et al., The therapeutic efficacy of adenoviral vectors for cancer gene therapy is limited by a low level of primary adenovirus receptors on tumor cells; Eur. J. Cancer 38 (2002), 1917-1926). Zudem hat sich herausgestellt, dass die Expression von CAR abhängig ist vom Differenzierungsgrad einer Zelle (Walters, R., et al., Adenovirus fiber disrupts CAR mediated intercellular adhesion allowing virus escape; Cell 110 (2002), 789-799). Dementsprechend ist die mit Adenoviren bisher erreichbare Transferrate heterologer Nucleinsäuren in Tumorzellen unbefriedigend niedrig.

Es ist daher versucht worden, den für die CAR-Erkennung maßgeblichen Abschnitt des Fiberknob-Proteins zu verändern, um den Viren ein Anbinden an andere Bestandteile der Zelloberfläche als lediglich den Coxsackie Adenovirus Rezeptor zu ermöglichen und somit den Tropismus der Viren zu verändern. Dieser Ansatz ist jedoch sehr aufwendig, da er eine Veränderung des Virengenoms erforderlich macht. Zudem ist die Kapazität des Fiberknob-Proteins für genetische Veränderungen sehr begrenzt (Suzuki, K., et al, A conditionally replicative adenovirus with enhanced infectivity shows improved oncolytic activity, Clin. Cancer Res. 7 (2001), 120-126).

Ebenfalls ist versucht worden, Fusionsproteine herzustellen, die die für die Fiberknobprotein-Erkennung verantwortliche extrazelluläre Domäne des Coxsackie Adenovirus Rezeptors und einen Ligandenabschnitt zur Erkennung durch einen auf der Oberfläche der Zielzelle exprimierten Rezeptor (Zielrezeptor) umfassen (Pereboev, A.V., et al., Coxsackievirus-adenovirus receptor genetically fused to anti-human CD40 scFv enhances adenoviral transduction of dendritic cells, Gene Ther. 9 (2002), 1189-1193). Dieser Ansatz erlaubt es, Zellen mit Adenoviren zu infizieren, die keinen oder nur in geringem Ausmaß den Coxsackie Adenovirus Rezeptor exprimieren, jedoch zumindest einen anderen Rezeptor exprimieren. Nachteilig ist jedoch, dass mit diesem Ansatz für jeden Zielrezeptor ein neues Fusionsprotein erzeugt werden muss, und dass die Infektion auf solche Zielzellen beschränkt bleibt, die den Zielrezeptor exprimieren (Curiel, D.T., Considerations and challenges for the achievement of targeted gene delivery, Gene Ther. 6 (1999), 1497-1498). Dies schränkt die Verwendbarkeit der Fusionsproteine insbesondere zur Infektion und Behandlung von Tumoren ein. Im Laufe ihrer Entwicklung werden Tumorzellen zunehmend genetisch instabil und ändern das Muster der von ihnen exprimierten Rezeptoren; dementsprechend kann jeder Tumorknoten über ein eigenes Spektrum an Zelloberflächenrezeptoren verfügen. Zur Tumorbehandlung wäre es deshalb erforderlich, zunächst die von allen Tumorzellen exprimierten Rezeptoren zu bestimmen, um anschließend entsprechende Fusionsproteine herzustellen.

Aufgabe der vorliegenden Erfindung war es daher, Mittel und Verfahren anzugeben, um auf möglichst einfache Weise das Spektrum der von Adenoviren infizierbaren Zellen (Zielzellen) zu erweitern. Vorteilhafterweise sollte die Infektion auch solcher Zielzellen mit einer annehmbaren oder guten Rate ermöglicht werden, die keinen oder nur in einem geringen Maße einen Coxsackie Adenovirus Rezeptor auf ihrer Zelloberfläche exprimieren. Die Mittel und Verfahren sollten ferner vorzugsweise sowohl therapeutisch, insbesondere zur Tumorbehandlung, als auch nicht-therapeutisch, insbesondere zur Infektion von Zellkulturen, verwendbar sein.

Darüber hinaus besteht auch allgemein ein Bedarf, eine Substanz auf möglichst einfache Weise an die Oberfläche einer Zielzelle ankoppeln zu können, wobei die Zielzelle aus einem möglichst großen Spektrum verschiedener Zelltypen auswählbar sein soll.

Erfindungsgemäß wird deshalb ein Adapter zum Ankoppeln einer an einer Zelloberfläche anzukoppelnden Substanz angegeben, umfassend:
a) einen Abschnitt zum Erkennen der und Binden an die anzukoppelnde Substanz, und
b) einen rekombinanten Abschnitt zum Anordnen des Adapters an der Zelloberfläche mit einer Affinität für eine oder mehrere negativ geladene Zelloberflächenstrukturen.

Ein erfindungsgemäßer Adapter kann gleichzeitig die anzukoppelnde Substanz erkennen und an sie binden, und gegebenenfalls durch Vermittlung weiterer Strukturen und Elemente der Zelloberfläche, insbesondere durch Vermittlung weit verbreiteter Rezeptoren, an der Zelloberfläche angeordnet werden. So kann auf vorteilhafte Weise weitgehend vermieden werden, für jeden Zelltyp einer ausgewählten Zielzelle ein an dessen spezifisches Rezeptor-Expressionsmuster angepasstes Fusionsprotein zu erzeugen.

Im Rahmen dieser Erfindung wird unter "Erkennen" die Fähigkeit des Adapters verstanden, mit einer im Schnitt höheren Affinität an die anzukoppelnde Substanz (insbesondere an das adenovirale Fiberknob-Protein) als an ein zufälliges anderes Protein zu binden. Das Erkennen und Binden des erfindungsgemäßen Adapters an die anzukoppelnde Substanz kann im Allgemeinen als Ligand-Rezeptor-Interaktion betrachtet werden, ist aber nicht auf einen solchen Mechanismus beschränkt.

Der Abschnitt b) des erfindungsgemäßen Adapters ermöglicht das Anordnen des Adapters an der Oberfläche der Zielzelle. Unter einem Anordnen des Adapters an der Oberfläche der Zielzelle wird dabei ein Verbinden der äußeren Zelloberfläche mit dem Adapter verstanden, wobei die Verbindung nicht lediglich eine zufällige und flüchtige ist. Stattdessen besitzt der erfindungsgemäße Adapter eine hohe Affinität zu der Zelloberfläche, insbesondere zu einigen außen auf der Zelloberfläche befindlichen Zelloberflächen-Elementen wie Proteinen und Kohlenhydraten.

Das Anordnen an der Zelloberfläche wird vorzugsweise durch Ankoppeln des erfindungsgemäßen Adapters an Zelloberflächen-Elemente bewirkt, die bei verschiedenen Zelltypen verbreitet oder ubiquitär sind. Insbesondere können die Zelloberflächen-Elemente Rezeptoren sein. Gemäß ersten Untersuchungen haben die negativ geladenen Elemente der Zelloberfläche, an die ein erfindungsgemäßer Adapter bindet, keine Signalweiterleitungsfunktion.

In bevorzugten Ausführungsformen hat der Abschnitt b) einen insgesamt basischen Charakter und/oder ist vorzugsweise positiv geladen. Peptide mit basischem Charakter und positiv geladene Peptide sind besonders gut zur dauerhaften Anordnung an Zellmembranen geeignet, ohne für diese Anordnung auf zelltypspezifische Elemente der Zelloberfläche angewiesen zu sein. Dabei können positiv geladene Peptide sich gut an negativ geladenen Strukturen der Zelloberfläche anlagern.

Vorteilhafterweise sind die Abschnitte a) und b) des Adapters miteinander kovalent verbunden, gegebenenfalls über einen verbindenden weiteren Abschnitt.

In bevorzugten Ausführungsformen umfasst der Abschnitt a) einen Abschnitt zum Erkennen des und Binden an das adenovirale Fiberknob-Protein.

Ein solcher Adapter kann gleichzeitig an ein herkömmliches adenovirales Fiberknob-Protein ankoppeln und durch Vermittlung weiterer Strukturen und Elemente der Zelloberfläche, insbesondere durch Vermittlung weit verbreiteter Rezeptoren, oder ohne Vermittlung weiterer Elemente der Zelloberfläche an der Zelloberfläche angeordnet werden. So kann auf vorteilhafte Weise vermieden werden, zum Erweitern des Spektrums der von Adenoviren infizierbaren Zellen die Adenoviren selbst genetisch verändern zu müssen; stattdessen können herkömmliche Adenoviren auch zum Infizieren bisher nicht oder nur schwer infizierbarer Zielzellen verwendet werden. Darüber hinaus umgeht die Erfindung durch Angeben des erfindungsgemäßen (rekombinanten) Adapters auch für viele Zelltypen die Notwendigkeit, für jeden zu infizierenden Zelltyp ein an dessen natives Rezeptor-Expressionsmuster angepasstes Fusionsprotein erzeugen zu müssen.

Besonders bevorzugt ist es, wenn der Abschnitt a) einen Teil der extrazellulären Domäne des Coxsackie Adenovirus Rezeptors (CAR) oder einen funktionsgleichen Teil umfasst.

Der Abschnitt a) eines solchen Adapters enthält einen Teil der extrazellulären Domäne des Coxsackie Adenovirus Rezeptors (gegebenenfalls also auch die vollständige extrazelluläre Domäne) oder einen dazu funktionsgleichen Teil. Funktionsgleich ist dabei ein Abschnitt a), der ein adenovirales Fiberknob-Protein erkennen, daran binden und somit den erfindungsgemäßen Adapter an das Fiberknob-Protein koppeln kann. Zur Auswahl des Abschnitts a) kann sich der Fachmann der bekannten Literatur betreffend den Aufbau und die Funktion des Coxsackie Adenovirus Rezeptors bedienen und entsprechend leicht eine Aminosäuresequenz finden, die zum Ankoppeln des Adapters an ein Fiberknob-Protein geeignet ist. Besonders bevorzugt als Abschnitt a) sind die Aminosäuren 1-235 des Coxsackie Adenovirus Rezeptors (Pubmed Accession No.: NM 001338, alle folgenden Accession-Nummern ebenfalls aus Pubmed).

Ferner kann der Fachmann neben oder anstelle eines Teils der extrazellulären Domäne des Coxsackie Adenovirus Rezeptors in Abschnitt a) einen Teil vorsehen, der zwar noch immer ein Ankoppeln des erfindungsgemäßen Adapters an das Fiberknob-Protein ermöglicht, jedoch nicht als solcher in der extrazellulären Domäne des Coxsackie Adenovirus Rezeptors enthalten ist. Insbesondere kann der Fachmann einzelne Aminosäuren der extrazellulären Domäne des Coxsackie Adenovirus Rezeptors weglassen, hinzufügen und/oder durch andere Aminosäuren ersetzen, solange die Funktion des Abschnitts a) erhalten bleibt, nämlich das Ankoppeln des erfindungsgemäßen Adapters an ein Fiberknob-Protein zu ermöglichen.

Mit dem erfindungsgemäßen Adapter ist es somit erstmals möglich, ohne detaillierte Vorkenntnisse über die von der Zielzelle exprimierten Rezeptoren einen Adapter mit hoher Affinität zu einem adenoviralen Fiberknob-Protein an der Oberfläche einer Zielzelle anzuordnen und diese somit für ein Ankoppeln eines Adenovirus-Partikels an die Zielzelle empfänglich zu machen. Der erfindungsgemäße Adapter ermöglicht auf vorteilhafte Weise, einen hohen Anteil von epithelialen Zellen, Fibroblasten, neuronalen Zellen, dendritischen Zellen, Osteoblasten und Myozyten sowie aus diesen Zelltypen hervorgegangene entartete Zellen mit Adenoviren zu infizieren.

Besonders bevorzugt ist ein Adapter, dessen Abschnitt b) ausgewählt ist aus:
- einem Peptid von 6 oder mehr Aminosäuren Länge, von denen zumindest die Hälfte Guanidino- und/oder Amidinoseitenketten enthalten,
- einem Oligoargininpeptid mit zumindest 6 Argininseitenketten,
- einem Peptid der Aminosäuresequenz entsprechend der basischen Region des HIV tat-Proteins,
- der dritten Helix eines Homöobox-Proteins, und
- einen die Verankerung an einer Zelloberfläche vermittelnden Abschnitt des Proteins VP22 des Herpes simplex-Virus.

Zwar ist bereits zuvor untersucht worden, ob Peptide der bevorzugten kennzeichnenden Art geeignet sind für ein Anordnen auf der Oberfläche eukaryontischer Zellen. Es ist jedoch nicht bekannt gewesen, diese Peptide mit einem Abschnitt zum Erkennen und Binden einer anzukoppelnden Substanz, insbesondere des adenoviralen Fiberknob-Proteins, zu versehen. Zudem wurde nunmehr gefunden, dass die genannten Peptide nicht nur zum Transport von Makromolekülen in Zellen geeignet sind, sondern auch (in Verbindung mit einem ein Adenovirus-Partikel erkennenden und bindenden Abschnitt a) eine für die Zwecke einer Infektion durch Adenoviren hinreichend dauerhafte Anlagerung des erfindungsgemäßen Adapters an der Zelloberfläche einer Zielzelle bewirken können.

So offenbart die US 6,495,663 ein Transportpolymer aus 6 bis 25 Untereinheiten, von denen zumindest 50% eine Guanidino- oder Amidino-Seitenkette enthalten. Das Transportpolymer ist geeignet, ein daran angehängtes Agens durch eine biologische Membran mit einer Rate zu transportieren, die größer ist als die des Agens als solchem. Zur Ausgestaltung des erfindungsgemäßen Adapters wird sich der Fachmann daher zweckmäßigerweise an der genannten US-Patentschrift orientieren, insbesondere sind die darin als bevorzugt gekennzeichneten Ausführungsformen auch im Rahmen der vorliegenden Erfindung bevorzugt. Als besonders wertvoll hat sich dabei ein Abschnitt b) herausgestellt, der linear ist, 7 bis 20 Aminosäuren lang ist, und wobei jede bis auf höchstens eine der Aminosäuren eine Guanidino- oder Amidino-Seitenkette besitzt.

Ferner offenbart die US 6,316,003 ein Transport-Polypeptid zum intrazellulären Ausliefern von "Fracht"-Molekülen mit Hilfe des HIV tat-Proteins oder eines oder mehrerer seiner Abschnitte. Als besonders nützlich wird darin die basische Region des tat-Proteins beschrieben, worunter insbesondere der Abschnitt der Aminosäuren 49 bis 57 verstanden wird. Der Fachmann wird sich zum Ausgestalten des erfindungsgemäßen Adapters daher gegebenenfalls an dieser Patentschrift orientieren. Die darin als bevorzugt genannten Ausführungsbeispiele sind deshalb auch erfindungsgemäß zur Ausgestaltung des Abschnitts b) des erfindungsgemäßen Adapters besonders bevorzugt. Hierzu zählt insbesondere ein Peptid mit der Aminosäuresequenz RKKRRQRRR.

Daneben offenbart die US 5,888,762 die Verwendung der dritten Helix eines Homöobox-Peptids zum Einführen eines Makromoleküls in eine lebende Zelle. Diese Patentschrift wird der Fachmann dementsprechend heranziehen, wenn er einen erfindungsgemäßen Adapter gemäß der vierten der oben aufgezählten Möglichkeiten herstellen will. Insbesondere sind erfindungsgemäß auch die in der US 5,888,762 als bevorzugt beschriebenen Ausführungsformen bevorzugt. Als erfindungsgemäß besonders vorteilhaft herausgestellt hat sich eine Homöobox-Proteinfragment mit den Aminosäuren 43 bis 58 der Homöodomäne, insbesondere aus dem Antennapedia-Homöoboxprotein; ebenfalls bevorzugt sind die diesen entsprechenden Abschnitte der Homöobox-Proteine von engrailed-1, engrailed-2, hoxa-5, hoxc-8 und fushi tarazu. Besonders bevorzugt umfasst der Abschnitt b) des erfindungsgemäßen Adapters ein Peptid mit der Aminosäuresequenz RQIKIWFQNRRMKWKK, also die Aminosäuresequenz der Aminosäuren 43-58 des Antennapedia Homeodomainproteins von Euprymna scolopes, Accession No. AY 052758.

Schließlich offenbart die US 6,184,038 die Verwendung von VP22 des Herpes simplex-Virus und dessen Homologen als Transportprotein. Auf die Offenbarung dieser Patentschrift wird der Fachmann entsprechend zurückgreifen, wenn er einen erfindungsgemäßen Adapter gemäß der letzten der oben aufgezählten Möglichkeiten herstellen will. Erfindungsgemäß besonders bevorzugt als Abschnitt b) des Adapters sind die 34 C-terminalen Aminosäuren des VP22-Proteins und entsprechende analoge und/oder homologe Peptide, soweit sie die Fähigkeit vermitteln, ein Protein an einer Zelloberfläche anzuordnen.

Insbesondere solche Adapter, bei deren Abschnitt b) die basische Region des HIV tat-Proteins und/oder einen die Verankerung an der Zelloberfläche vermittelnden Abschnitt des VP22-Proteins des Herpes simplex-Virus bzw. einen Teil dieser Peptide umfasst, können an negativ geladene Elemente der Zelloberfläche von Zellen unterschiedlicher Genese anlagern. Besonders gut gelingt die Anlagerung an die Zelloberfläche von Zellen epithelialen Ursprungs. Gemäß den bisherigen Untersuchungen gelingt die Anlagerung ebenfalls gut an die Zelloberflächen von Zellen, die stark zur Zelladhäsion neigen. Damit sind die erfindungsgemäßen Adapter vorteilhaft geeignet zum Herstellen eines Arzneimittels zur Bekämpfung von Tumoren epithelialen Ursprungs und/oder zur Bekämpfung von Tumoren mit starker Neigung zur Zelladhäsion. Dies gilt insbesondere für epitheliale Karziome, Sarkome, Glioblastome und Astrozytome.

Es hat sich ferner als nützlich herausgestellt, wenn der erfindungsgemäße Adapter zusätzlich einen Oligomerisierungsabschnitt zum Bilden von Di-, Triund/oder Oligomeren des Adapters umfasst. Durch das Vorsehen des Oligomerisierungsabschnitts kann die Affinität des erfindungsgemäßen Adapters zum adenoviralen Fiberknob-Protein bzw. der anzukoppelnden Substanz auf einfache Weise dauerhaft gesteigert werden. Als Oligomerisierungsabschnitt ist insbesondere die Oligomerisierungsdomäne des GCN4-Proteins und/oder Leucin-Zipper-Domänen bevorzugt.

Besonders gut gelang die Herstellung des erfindungsgemäßen Adapters, wenn dieser ferner eine Leadersequenz umfasst, um die Synthese des Adapters in das rauhe endoplasmatische Retikulum und vorzugsweise auch in den Extrazellularraum zu bewirken. Auf diese Weise kann der erfindungsgemäße Adapter leicht aus einer diesen herstellenden eukaryontischen Zelle ausgeschleust werden. Als Leadersequenz besonders bevorzugt ist die natürliche Leadersequenz des Coxsackie Adenovirus Rezeptors. Zweckmäßigerweise besitzt der erfindungsgemäße Adapter keine Transmembrandomäne des Coxsackie Adenovirus Rezeptors. Durch das Fehlen dieser Transmembrandomäne wird das Abgeben des erfindungsgemäßen Adapters aus einer exprimierenden eukaryontischen Zelle in den Extrazellularraum zusätzlich vereinfacht.

Erfindungsgemäß wird weiter eine Nucleinsäure angegeben mit einem Abschnitt, der für einen Adapter nach einer der zuvor beschriebenen erfindungsgemäßen Arten codiert. Eine solche Nucleinsäure vereinfacht das Herstellen des Adapters in einer Wirts- oder Produktionszelle.

Die für den Adapter kodierende Nucleinsäure kann insbesondere Teil eines Virusgenoms sein und darin vorzugsweise in Form einer Expressionseinheit vorliegen. Dementsprechend wird erfindungsgemäß auch ein rekombinantes Virus angegeben mit gegenüber der Wildtypform verbesserten Ausbreitungseigenschaften in einer Kultur eukariontischer Zellen, insbesondere menschlicher und/oder tierischer Zellen. Das Virus kann insbesondere auch gegenüber seiner Wildtypform verbesserte Tumor-Lyseeigenschaften besitzen.

Das Virus zum Exprimieren eines Adapters nach einer der zuvor beschriebenen erfindungsgemäßen Arten besitzt in bevorzugten Ausführungsformen zumindest in einem Stadium seiner Vermehrung eine Virushülle, und es enthält:
- eine Nucleinsäure wie soeben beschrieben, und
- einen Nucleinsäure-Abschnitt zur Expression eines Proteins der Virus-Hülle, an das der Abschnitt a) des Adapters ankoppeln kann.

Dabei wird unter einem Virus eine nucleinsäurehaltige Einheit verstanden, deren Nucleinsäure in einer Wirtszelle vermehrbar ist und wobei die Nucleinsäure in ein gegebenenfalls aus mehreren Einheiten zusammengesetztes Partikel (Virus-Partikel) verpackt werden kann, und wobei die gegebenenfalls in ein Virus-Partikel verpackte Nucleinsäure aus der Wirtszelle (beispielsweise beim Tod der Wirtszelle) freigesetzt werden kann, um in eine weitere Wirtszelle einzudringen. Ein Beispiel für ein erfindungsgemäßes Virus ist ein von einem Adenovirus des Typs 2 und/oder 5 abgeleitetes Virus mit einer Nucleinsäure zur Expression eines erfindungsgemäßen Adapters, dessen Abschnitt a) einen Abschnitt zum Erkennen des und Binden an das adenoviral Fiberknob-Protein enthält.

Unter einem Adenovirus-Partikel (in dieser Beschreibung gelegentlich auch als "Virus-Partikel" abgekürzt) wird jeder Körper verstanden, der eine Nucleinsäure einschließt oder auf andere Weise mit ihr gekoppelt ist, die, wenn sie exprimiert wird, seine eigene Herstellung bewirkt, und zumindest ein adenovirales Fiberknob-Protein besitzt. Insbesondere sind die Virus-Partikel herkömmlicher Adenoviren Adenovirus-Partikel im Sinne der Erfindung. Es ist ferner besonders bevorzugt, wenn das Adenovirus-Partikel ein Virus-Partikel eines erfindungsgemäßen Virus wie soeben beschrieben ist.

Die erfindungsgemäßen Viren ermöglichen auf vorteilhaft einfache Weise, in einer Wirtszelle sowohl einen erfindungsgemäßen Adapter herzustellen, als auch eine Virus-Hülle bzw. ein Virus-Partikel herzustellen, das von dem erfindungsgemäßen Adapter erkannt und gebunden werden kann. Das erfindungsgemäße Virus kann sich dann in einer Kultur von Zielzellen auszubreiten, ohne dass diese Kultur nativ über besondere Rezeptoren zum Ankoppeln des Virus an die Zielzellen verfügen müsste. Sobald die erste Zielzelle infiziert ist und die Herstellung des erfindungsgemäßen Adapters eingesetzt hat, wird dieser aus der ersten Zielzelle freigesetzt und auf der Zelloberfläche weiterer Zielzellen angeordnet. Aus der ersten Zielzelle freigesetzte erfindungsgemäße Viren können dann an die so veränderten weiteren Zielzellen über die erfindungsgemäßen Adapter ankoppeln und diese weiteren Zielzellen infizieren.

Entsprechend besonders bevorzugt ist ein Virus, dessen Vermehrung gewebespezifisch reguliert ist. Auf diese Weise kann die Ausbreitung der erfindungsgemäßen Viren im wesentlichen auf bestimmte Gewebe (Zielgewebe), beispielsweise Tumorgewebe, begrenzt werden. Bevorzugte gewebespezifisch regulierte Promotoren, die zur gewebespezifischen Regulierung der Virus-Vermehrung verwendet werden können, sind: Promotoren für den AFP-(Alphafetoprotein)-Tumormarker, für das prostataspezifische Antigen (PSA), für die Proteinuntereinheit der Telomerase (hTERT), sowie Promotoren, die durch Transkriptionsfaktoren reguliert werden, insbesondere durch die Transkriptionsfaktoren myc, myb, E2F und solche Transkriptionsfaktoren, die infolge mitogener Signale aktiviert und/oder verstärkt exprimiert werden.

Gleichzeitig oder alternativ dazu ist ein Virus bevorzugt, dessen für den erfindungsgemäßen Adapter codierender Nucleinsäureabschnitt gewebespezifisch reguliert ist. Auf diese Weise kann das Exprimieren des erfindungsgemäßen Adapters im wesentlichen auf ein Zielgewebe begrenzt werden. Zur gewebespezifischen Regulation können wiederum die im vorangegangenen Absatz beschriebenen Promotoren verwendet werden.

Ebenfalls bevorzugt sind solche Viren, die ferner eine Nucleinsäure umfassen, die für ein zelltodauslösendes Mittel codiert, wobei es besonders bevorzugt ist, wenn die Nucleinsäure eingerichtet ist, das zelltodauslösende Mittel in gewebespezifischer Weise zu exprimieren. Die entsprechende Nucleinsäure kann insbesondere ein Abschnitt der Nucleinsäure sein, die den für den erfindungsgemäßen Adapter codierenden Abschnitt enthält. Bevorzugte zelltodauslösende Mittel, für die eine erfindungsgemäße Nucleinsäure codieren kann, sind die Herpes simplex-Virus-Thymidinkinase und die Cytosindeaminase. Insbesondere ist es bevorzugt, wenn das Virus onkolytisch ist.

Wenn der erfindungsgemäße Adapter eine höhere Affinität zum Virus-Partikel, insbesondere zum Fiberknob-Protein, als zur Zelloberfläche bzw. zu dem/den die Anordnung des Adapters an der Zelloberfläche vermittelnden Elementen der Zelloberfläche besitzt, wird ein Virus-Partikel gewöhnlich zunächst mit mehreren erfindungsgemäßen Adaptern ummantelt, bevor es an einer Zelloberfläche angelagert wird.

Zudem wird erfindungsgemäß die Verwendung eines erfindungsgemäßen Adapters zum nicht-therapeutischen Vermitteln und/oder Verbessern des Ankoppelns einer Substanz, insbesondere eines Adenovirus-Partikels, an eine Zelloberfläche gelehrt. Wie oben beschrieben ermöglicht es der erfindungsgemäße Adapter, im wesentlichen unabhängig von nativen Rezeptoren oder unter Vermittlung verbreiteter Zelloberflächen-Elemente einer Wirtszelle diese für eine Infektion mit Adenovirus-Partikeln oder für ein Ankoppeln einer anderen anzukoppelnden Substanz empfänglich zu machen. Auf diese Weise lässt sich die Transformationseffizienz beispielsweise bei der Infektion einer eukaryontischen Zellkultur vorteilhaft einfach verbessern.

Die erfindungsgemäßen Adapter eignen sich besonders gut, die Infektionsrate solider Tumore mit Adenoviren zu erhöhen. Auf diese Weise kann der zur Infektion benötigte Virus-Titer verringert werden, wodurch im Tiermodell oder auch beim Menschen die Lebertoxizität eines Adenoviren enthaltenden Medikaments oder Präparats verringert werden kann.

Dementsprechend wird erfindungsgemäß auch ein Verfahren angegeben zum nicht-therapeutischen Ankoppeln einer Substanz, insbesondere eines Adenovirus-Partikels, an eine Zelloberfläche, umfassend die Schritte:
a) Exponieren der anzukoppelnden Substanz (insbesondere des Adenovirus-Partikels) gegen einen erfindungsgemäßen Adapter, um die Substanz mit dem Adapter zu versehen, und
b) Exponieren der Zelloberfläche gegen die mit dem Adapter versehene, anzukoppelnde Substanz.

In Schritt a) kann insbesondere ein Adenovirus-Partikel mit dem erfindungsgemäßen Adapter versehen werden. Dabei ist es vorteilhaft, das Virus-Partikel mehreren erfindungsgemäßen Adaptern auszusetzen, um das Virus-Partikel mit dem erfindungsgemäßen Adapter zu ummanteln. Im zweiten Schritt wird die Zielzelle einer Dosis der so behandelten Adenovirus-Partikeln (zumindest 1 Partikel) ausgesetzt, um ein Ankoppeln zumindest eines Partikels an die Zelloberfläche über den erfindungsgemäßen Adapter zu ermöglichen. Auf diese Weise wird erstmals eine effektive Ankopplung von Adenoviren an Zielzellen ermöglicht, die keinen oder nur eine geringe Menge des Coxsackie Adenovirus Rezeptors auf ihrer Zelloberfläche exprimieren und entsprechend bisher schlecht mit Adenoviren infizierbar waren. Beispielsweise ist es mit dem erfindungsgemäßen Verfahren erstmals möglich, mit zufriedenstellender Effizienz Stroma-Fibroblasten zu infizieren.

In einem therapeutischen Verfahren kann ein erfindungsgemäßer Adapter ebenfalls eingesetzt werden zum Vermitteln und/oder Verbessern des Ankoppelns einer Substanz, insbesondere eines Adenovirus-Partikels, an eine Zelloberfläche. Letzteres ist insbesondere im Rahmen von Gentherapie-Verfahren mit Adenoviren und/oder daraus abgeleiteten Viren, beispielsweise zur Tumorbekämpfung, von Vorteil. In einem therapeutischen Verfahren ist es ebenfalls bevorzugt, zunächst ein Virus-Partikel mit dem erfindungsgemäßen Adapter zu versehen (insbesondere zu ummanteln), und anschließend eine Zielzelle gegen das so behandelte Virus-Partikel zu exponieren.

Es ist daher bevorzugt, eine erfindungsgemäße Nucleinsäure und/oder ein erfindungsgemäßes Virus zu verwenden zum Herstellen eines Arzneimittels zum Vermitteln und/oder Verbessern des Ankoppelns einer Substanz, insbesondere eines Adenovirus-Partikels, an eine Zelloberfläche, wobei die Verwendung eines erfindungsgemäßen Virus besonders bevorzugt ist. Mit einem solchen Arzneimittel lassen sich die mit der soeben beschriebenen therapeutischen Verwendung der erfindungsgemäßen Adapter und Viren erzielbaren Vorteile besonders einfach verwirklichen. Insbesondere eröffnet ein entsprechendes Arzneimittel erstmals die Möglichkeit, auch bisher nicht oder nur schwer mit Adenoviren infizierbare Zellen, insbesondere Tumorzellen und Stroma-Fibroblastenzellen, in einem für therapeutische Zwecke ausreichendem Maße zu infizieren. Fibroblastenzellen besitzen eine wesentliche Rolle für die Ernährung wachsender Tumore, das Verbessern ihrer Infizierbarkeit stellt daher einen erheblichen Fortschritt in der Tumor-Therapie dar.

Ein erfindungsgemäßes Arzneimittel zum Vermitteln und/oder Verbessern des Ankoppelns einer Substanz (insbesondere eines Adenovirus-Partikels) an eine Zelloberfläche umfasst deshalb
- einen erfindungsgemäßen Adapter und/oder ein erfindungsgemäßes Virus, und
- einen pharmazeutisch akzeptablen Träger.

Zweckmäßigerweise kann es sich bei dem Arzneimittel um ein aus zwei getrennten Teilen bestehendes Präparat handeln, dessen einer Teil einen erfindungsgemäßen Adapter und dessen anderer Teil ein erfindungsgemäßes Virus umfasst. Soll das Arzneimittel zum Vermitteln und/oder Verbessern des Ankoppelns eines Adenovirus-Partikels dienen, so ist es bevorzugt, vor dem Verabreichen an einen Patienten die beiden Teile des Arzneimittels miteinander zu mischen, um die adenoviralen Fiberknob-Proteine mit dem erfindungsgemäßen Adapter zu koppeln; die so entstehenden adapterbeladenen Viren sind besonders geeignet zum Infizieren im wesentlichen beliebiger Gewebe, insbesondere auch solcher, die keinen oder nur eine geringe Menge des Coxsackie Adenovirus Rezeptors exprimieren.

Die oben beschriebenen erfindungsgemäßen Verfahren und Verwendungen sind insbesondere einsetzbar im Zusammenhang mit Zellen menschlichen Ursprungs. Die erfindungsgemäßen Adapter sind jedoch auch geeignet, das Anordnen einer Substanz, beispielsweise eines adenoviralen Fiberknob-Proteins (einschließlich eines dieses Protein umfassenden Virus-Partikels), an der Oberfläche einer Zielzelle sonstigen Ursprungs zu bewirken, soweit der Abschnitt b) des Adapters so ausgewählt ist, dass dieser an der Oberfläche der Zielzelle angeordnet werden kann. Eine Zielzelle sonstigen Ursprungs kann insbesondere eine Zielzelle aus bzw. in einem Affen oder Hund sein.

Die Erfindung wird nachfolgend anhand der Beispiele und der Figuren näher erläutert, wobei die Beispiele den Gegenstand der Erfindung nicht begrenzen sollen. Es stellen dar:
- Fig. 1: schematischer Aufbau erfindungsgemäßer Adapter;
- Fig. 2: Photographien von Adenovirus-infizierten Zellkulturen, wobei das Adenovirus mit einem erfindungsgemäßen Adapter ummantelt worden ist;
- Fig. 3: schematische Darstellung der Wirkungsweise erfindungsgemäßer Adapter;
- Fig. 4: weitere schematische Darstellung der Wirkungsweise erfindungsgemäßer Adapter.

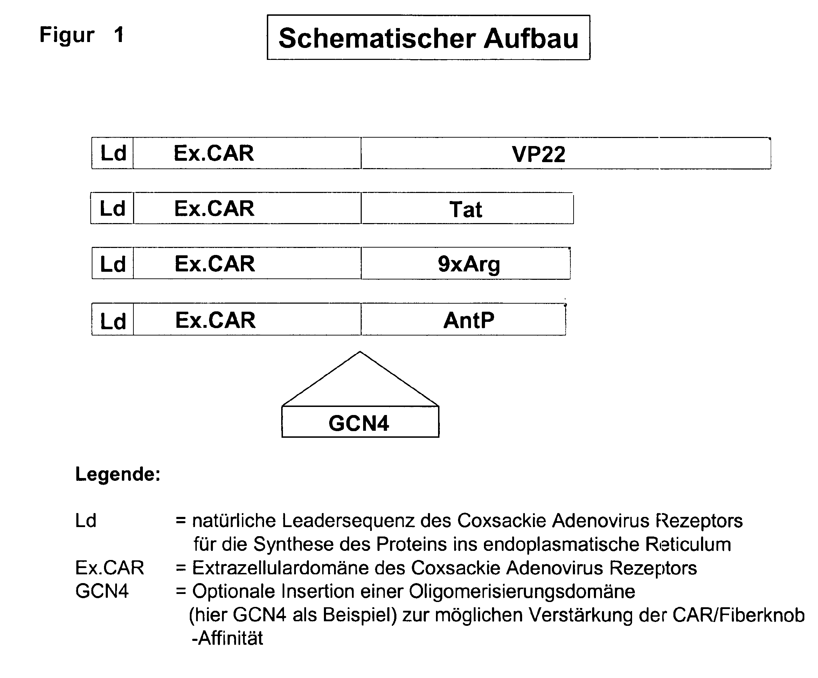

Fig. 1 zeigt schematisch den Aufbau von vier erfindungsgemäßen Adapter-Proteinen in ihrer Primärstruktur. Alle Adapter besitzen am N-Terminus eine Leadersequenz des Coxsackie Adenovirus-Rezeptors. Die Leadersequenz bewirkt die Synthese des erfindungsgemäßen Adapters in das endoplasmatische Retikulum einer den Adapter exprimierenden Wirtszelle. An die Leadersequenz schließt sich die Extrazellulardomäne des Coxsackie Adenovirus-Rezeptors an, sie entspricht Abschnitt a) des erfindungsgemäßen Adapters. Auf die Extrazellulardomäne folgt in C-terminaler Richtung der Abschnitt b) des erfindungsgemäßen Adapters. Dieser ist in den vier dargestellten Adapter (von oben nach unten) jeweils ausgeführt als ein die Verankerung an einer Zelloberfläche vermittelnder Abschnitt des Proteins VP22 des Herpes simplex-Virus, die basische Region des HIV tat-Proteins, ein Oligoargininpeptid mit 9 Argininresten und die dritte Helix eines Homöobox-Proteins. Zwischen den Abschnitten a) und b) kann, wie dargestellt, eine die Dimerisierung fördernde Domäne (hier: der Leucin-Zipper aus GCN4) angeordnet sein; sie kann die Affinität des erfindungsgemäßen Adapters zum Fiberknob-Protein erhöhen.

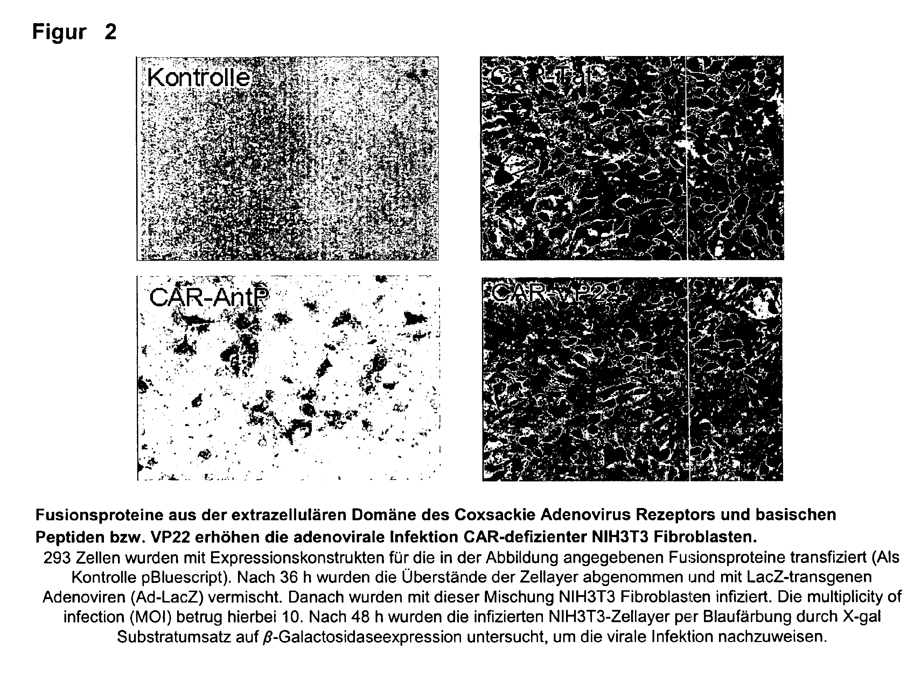

Fig. 2 zeigt Photographien von Zellkulturen von NIH3T3-Zellen, die mit Adenoviren unter Verwendung erfindungsgemäßer Vektoren infiziert worden sind. Als Kontrolle (oben links dargestellt) diente eine Kultur von NIH3T3-Zellen, die gegen Adenoviren ohne erfindungsgemäßen Vektor exponiert wurde. Die erfindungsgemäßen Adapter wurden vor der Infektion der NIH3T3-Zellen in 293-Zellen exprimiert und in den Zellüberstand ausgeschieden. Der Zellüberstand wurde 36 Stunden nach der Transfektion der 293-Zellen mit einer für den jeweiligen erfindungsgemäßen Adapter codierenden Nucleinsäure abgenommen und mit LacZ-codierenden Adenoviren vermischt. Die so mit einem jeweiligen erfindungsgemäßen Adapter versehenen Adenoviren wurden zu einer Kultur NIH3T3-Zellen gegeben, um diese zu infizieren. Die multiplicity of infection (MOI) betrug 10. Nach 48 Stunden wurden die Zellkulturen durch Xgal-Blaufärbung auf β-Galactosidaseexpression untersucht. Zellen, die mit einem Adenovirus infiziert waren, wurden dabei blau gefärbt. Die Infektion gelang am besten mit solchen erfindungsgemäßen Adapter, die entweder die basische Region des HIV tat-Proteins (oben rechts dargestellt, "CAR-Tat") oder ein die Verankerung an einer Zelloberfläche vermittelnder Abschnitt des Proteins VP22 des Herpes simplex-Virus (unten rechts dargestellt, "CAT-VP22") im Abschnitt b) besaßen. Aber auch ein erfindungsgemäßer Adapter mit der dritten Helix des AntP-Homöobox-Proteins im Abschnitt b) führte zu einer gegenüber der Kontrolle deutlich verbesserten Infektionsrate (unten links dargestellt, "CAR-AntP").

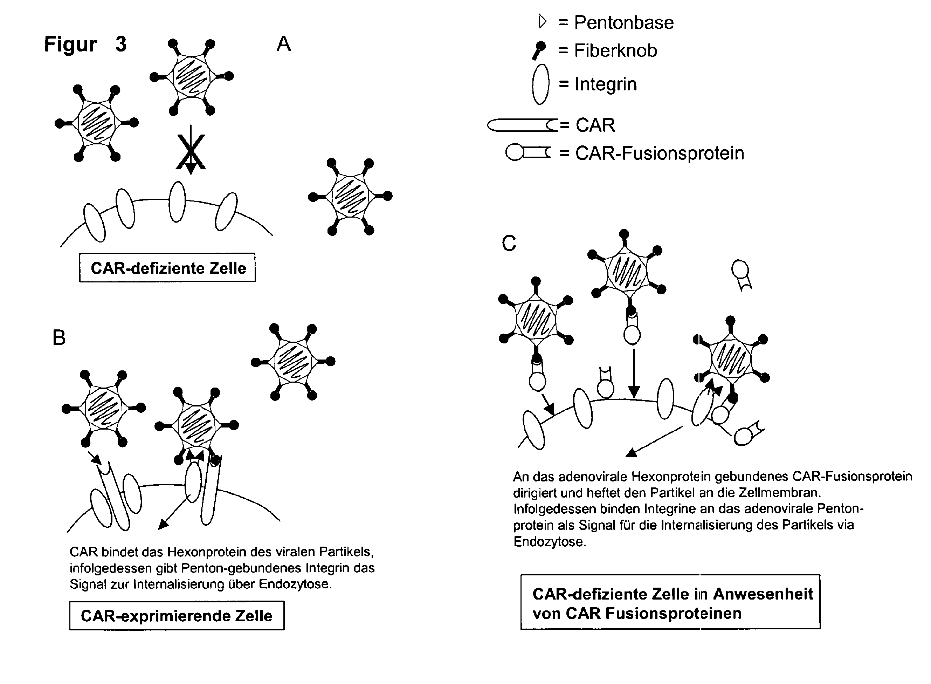

Fig. 3 zeigt schematisch die Wirkungsweise erfindungsgemäßer Adapter. Adenovirus-Partikel können CAR-defiziente Zellen nicht oder nur schlecht infizieren (Darstellung oben links). Die Infektionsrate wird deutlich gesteigert, wenn die zu infizierende Zelle auf ihrer Zelloberfläche den Coxsackie Adenovirus-Rezeptor exprimiert (unten links dargestellt). Ein erfindungsgemäßer Adapter vermittelt die Anhaftung eines Adenovirus-Partikels an eine CAR-defiziente Zelle (Darstellung rechte Hälfte). Der erfindungsgemäße Adapter bindet dazu mit seinem Abschnitt a) an ein Fiberknob-Protein des Adenovirus-Partikels. Sein Abschnitt b) besitzt eine hohe Affinität zur Zelloberfläche der Zielzelle, insbesondere zu bei dem Zelltyp der Zielzelle weit verbreiteten Elementen der Zelloberfläche. Der erfindungsgemäße Adapter vermittelt den Kontakt zwischen der Zelloberfläche bzw. den Elementen der Zelloberfläche, zu denen der Adapter eine hohe Affinität besitzt, und dem Adenovirus-Partikel. Nach Herstellen des Kontakts kann das Adenovirus-Partikel internalisiert und die Infektion der Zielzelle vollzogen werden.

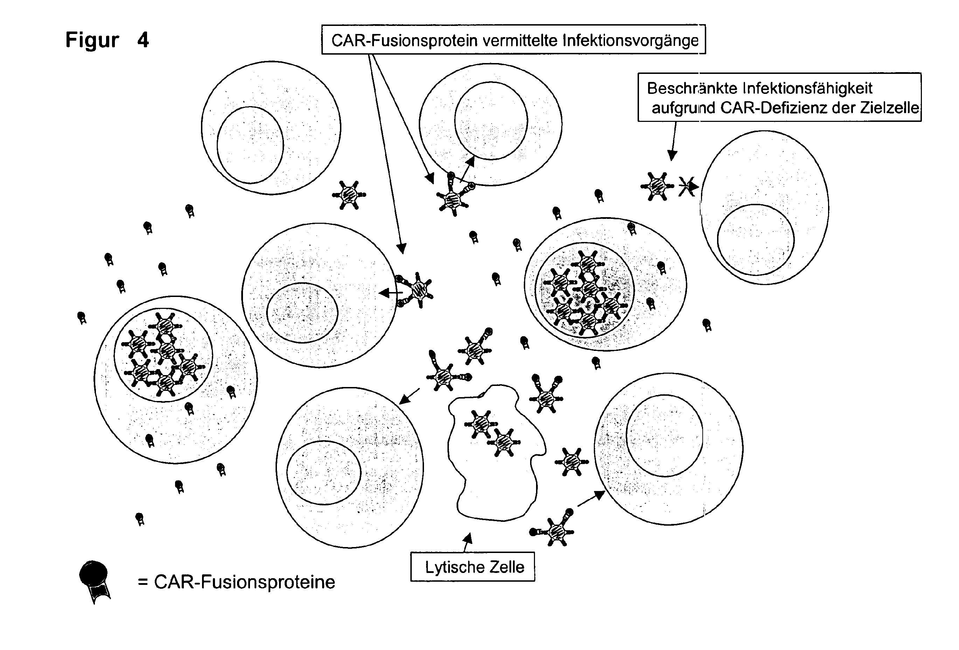

In Fig. 4 ist schematisch eine Zellkultur dargestellt, die teilweise mit Adenoviren infiziert ist (Zelle links außen und rechts Mitte), wobei die Adenoviren auch für einen erfindungsgemäßen Adapter codieren. Die infizierten Zellen exprimieren sowohl den erfindungsgemäßen Adapter und setzen ihn in den Extrazellularraum frei. Sie produzieren jedoch auch neue Adenovirus-Partikel; diese werden bei der Zellyse (unten Mitte dargestellt) freigesetzt und von im Extrazellularraum befindlichen erfindungsgemäßen Adapter teilweise ummantelt. Die so (zumindest teilweise) ummantelten Adenoviren können eine weitere Zelle infizieren (dargestellt links Mitte, oben Mitte und unten rechts), um dort sowohl den erfindungsgemäßen Adapter als auch neue Adenovirus-Partikel herstellen zu lassen. Die Infektion der Zellkultur breitet sich so selbständig aus.

### Beispiel 1: Herstellung der Polynucleotide

Die für die Generierung eines CAR₁₋₂₃₅-VP22-Fusionsproteins (ein erfindungsgemäßer Adapter) notwendige cDNA wurde mittels PCR generiert. Als Template diente die im Plasmid LXSN-hCAR (freundliche Überlassung von Dr. DeGregori, Denver, USA) enthaltene cDNA des humanen Coxsackievirus und Adenovirusrezeptors (hCAR). Als 5'-Primer wurde das Oligonukleotid 5'-GT*GGTACC* **ATG** GCG CTC CTG CTG TGC TTC GTG C-3' (Kpn I-Schnittstelle kursiv, natürliches Startcodon von hCAR in Fettdruck) und als 3'- Primer das Oligonukleotid 5'-TA*GCGGCCGC***C TTT ATT TGA AGG AGG GAC AAC GTT TAG ACG C**-3' verwendet (Not I-Schnittstelle kursiv, Komplementäre der Basen 776 bis 807 von hCAR (Entrez Pubmet Accession No.: NM 001338) in Fettdruck). Das entstehende Fragment enthält die Leadersequenz von hCAR sowie dessen Extrazellulardomäne. In der PCR wurden 5 Zyklen bei 49°C und 30 Zyklen bei 55°C unter Verwendung von Pfu Polymerase durchgeführt. Das PCR-Produkt wurde mithilfe einer Agarosegelelektrophorese aufgereinigt und die Enden durch Verdau mit Kpn I und Not I regeneriert. Dieses Fragment wurde anschließend in die entsprechenden Restriktionsschnittstellen des Vektors pVP22mycHis 2 (Invitrogen) eingefügt, resultierend in das Plasmid pCAR(ex)-VP22. Die Plasmide für Fusionsproteine der Extrazellulardomäne von hCAR mit den Zelladhäsionsdomänen von HIV-TAT, AntP bzw. einer 9 x Argininsequenz (9xArg) wurden wie folgt kloniert:

Zur Generierung von pCAR(ex)-TAT₄₈₋₅₇ wurden das Oligonukleotid 5' AAA*GC GGC CGC* GGA GGA GGA AGT GGA GGA GGA GGA **GGC AGG AAG AAG CGG AGA CAG CGA CGA AGA** *GGT CTA GA*AA-3' (sense-Oligonukleotid, Fettdruck entspricht den Basen 5518-5547 des HIV-Genoms, Accession No.: NC 001802, kodierend für die Aminosäuren 48-57 (Sequenz: GRKKRRQRRR) des TAT-Proteins, Schnittstellen für Not I und Xba I kursiv) und ein entsprechend komplementäres antisense Oligonukleotid (5'-TTTCTAGACCTCTTCGTCGCTGTCTCCGCTTCTTCCTGCCTCCTCCTCCT CCACTTCCTCCTCCGCGGCCGCTTT-3') hybridisiert, mit Not I und Xba I verdaut und in die entsprechenden Schnittstellen des Plasmids pCAR(ex)-VP22 eingefügt. Dieses wurde zuvor mit den Restriktionsenzymen Not I und Xba I geschnitten und die für VP22 kodierende cDNA durch Agarosegelelektrophorese abgetrennt.

Die Generierung der AntP und 9xArg Varianten erfolgte durch vergleichbare Arbeitsschritte.

Als für 9xArg kodierende DNA wurde das Oligonukleotid 5'AAAGC GGC CGC GGA GGA GGA AGT GGA GGA GGA GGA **CGT CGC CGA CGG AGA AGG AGA CGT AGA** GG*T CTA GA*AA-3' (sense-Oligonukleotid, Fettdruck entspricht der für die Aminosäuresequenz RRRRRRRRR kodierenden DNA, Schnittstellen für Not I und Xba I kursiv) sowie ein entsprechend komplementäres antisense Oligonukleotid verwendet 5'-TTTCTAGACCTCTACGTCTCCTTCTCCGTCGGCGACGTCCTCCTCCTC CACTTCCTCCTCCGCCGCGGCCGCTTT-3'. Name des resultierenden Plasmids: pCAR(ex)-9xArg.

Als für AntP kodierende DNA wurde das Oligonukleotid 5'AAGC GGC CGC GGA GGA GGA GGA **AGA CAG ATC AAA ATA TGG TTC CAA AAC CGG CGC ATG AAA TGG AAG AAA** *GGT CTA GA*AA-3' (sense-Oligonukleotid, Fettdruck entspricht den Basen 184-231 der partialen cds für Euprymna scolopes antennapedia homeodomain protein, Accession No.: AY052758, kodierend für die Aminosäuren 62-77, Sequenz: RQIKIWFQNRRMKWKK, Schnittstellen für Not I und Xba I kursiv) sowie ein entsprechend komplementäres antisense Oligonukleotid verwendet 5'-TTTCTAGACCTTTCTTCCATTTCATGCGCCGGTTTTGGAACCATATTTT GATCTGTCTTCCTCCTCCTCCGCGGCCGCTT-3'. Name des resultierenden Plasmids: pCAR(ex)-AntP₆₂₋₇₇.

Alle verwendeten Oligonukleotide verfügen in 5'-Richtung der Zelladhäsionsdomäne noch über mehrere Glycin/Serinreste, um diese Domäne von der CAR-Domäne räumlich zu trennen.

Die oben genannten Plasmide wurden sequenziert und mit dem Qiagen Endofree Plasmid Maxi Prep Kit präpariert. Die Produktion analytischer Mengen von CAR-Fusionsproteinen erfolgte durch Transfektion von 293-Zellen mit der Kalziumpräzipitationsmethode bzw. Liposomenbildnern. 24-48 h nach der Transfektion konnten die in den Zellkulturüberständen enthaltenen Fusionsproteine für analytische Zwecke weiterverwendet werden.

### Beispiel 2: Herstellung der für CAR(ex)-Fusionsproteine kodierenden Adenoviren für die präparative Proteinproduktion aus infizierten Zellkulturen

Die Generierung des Adenovirus, der eine Expressionseinheit für CAR₁₋₂₃₅-VP22 enthält, erfolgte mit Hilfe des Klonierungssystems nach Mizuguchi und Kay (Hum. Gene Ther.1998, 9(17): 2577-83). Als Ausgangspunkt für den Shuttlevektor wurde eine modifizierte Variante des Vektors pHM3 verwendet (pHM3/pBK-CMV), der in der MCS von pHM3 die komplette Expressionskassette (CMV-Promoter/MCS/SV40-Polyadenylierungssignal) des Vektors pBK-CMV (Stratagene) enthält. Die cDNA für CAR₁₋₂₃₅-VP22 wurde aus dem Plasmid pCAR(ex)-VP22 durch Hind III Verdau, Behandlung mit Klenow-Fragment und anschließendem Pme I Verdau präpariert und in die Sma I Schnittstelle des Vektors pHM3/pBK-CMV eingefügt. Die korrekte Orientierung des resultierenden Konstruktes (p4622) wurde durch Kpn I Verdau überprüft. Die Klonierung des adenoviralen Plasmids erfolgte durch Isolierung des PI-Sce/I-Ceu Fragmentes aus p4622 und dessen Ligation in die entsprechenden Schnittstellen des adenoviralen Vektors pAdHM4. Die Ligation resultierte in das Konstrukt pAd4679. Mit einer Pac I linearisierten Präparation dieses Vektors wurden 293-Zellen transfiziert, um infektiöse Partikel zu generieren. Nach Auftreten eines cytopathischen Effektes wurden die Zellen geerntet und die infektiösen Partikel durch dreimaliges Einfrieren und Auftauen freigesetzt. Der Zelldetritus wurde abzentrifugiert und der Überstand zur Herstellung höhertitriger Viruspräparationen eingesetzt (Bezeichnung des gewonnenen Adenovirus: Ad4679). Die Gewinnung von höheren Titern rekombinanter Viren erfolgte ebenfalls in 293 Zellen und der anschließenden Aufreinigung virushaltiger Lysate über CsCl-Gradienten-Ultrazentrifugation.

Die Generierung des Adenovirus mit einer Expressionskassette für CAR₁₋₂₃₅-TAT₄₈₋₅₇ erfolgte mit Hilfe des Rekombinationssystems von He und Vogelstein (He et al., Proc. Natl. Acad. Sci. USA 1998, 95: 2509-2514). Die für CAR₁₋₂₃₅-TAT₄₈₋₅₇ kodierende cDNA wurde aus dem Plasmid pCAR(ex)-TAT durch Hind III und Pme I Verdau isoliert und in die Hind III und EcoR V Schnittstellen des Vektors pShuttle-CMV ligiert. Die Ligation resultierte in das Konstrukt p4923. Das Konstrukt p4923 wurde mit Pme I linearisiert und anschließend mit dem adenoviralen Vektor AdEasy 1 gemischt. Mit diesem Ansatz wurden rekombinationsfähige BJ5183 E. coli elektroporiert. Das hierdurch generierte rekombinante Adenovirusplasmid wurde als p5091 bezeichnet. Mit einer Pac I verdauten Präparation dieses Vektors wurden 293-Zellen transfiziert, um infektiöse Partikel zu generieren. Nach Auftreten eines cytopathischen Effektes wurden die Zellen geerntet und die infektiösen Partikel durch dreimaliges Einfrieren und Auftauen freigesetzt. Der Zelldetritus wurde abzentrifugiert und der Überstand zur Herstellung höhertitriger Viruspräparationen eingesetzt (Bezeichnung des gewonnenen Adenovirus: Ad5091).

### Beispiel 3: Herstellung der rekombinanten CAR-Fusionsproteine aus den Überständen adenoviral infizierter Zellen

Die rekombinanten CAR(ex)-Fusionsproteine verfügen an ihrem C-Terminus über ein 6 x Histidin Motiv, welches positiv geladene Ni-lonen zu komplexieren vermag. Auf diese Weise können die Fusionsproteine mit Hilfe von festphasengebundenen Nickelionen aus komplexen Proteinmischungen aufgereinigt werden.

Die zuvor beschriebenen Adenoviren Ad4679 und Ad5091 wurden als Vektoren für die Produktion der CAR-Fusionsproteine in infizierten Wirtszellen verwendet.

Als Zellinien für die Produktion der rekombinanten Proteine eignen sich prinzipiell alle adhärenten, adenoviral infizierbaren Zelltypen, wie z. B. COS-1, HepG2, Huh 7 etc. Infizierte COS-1 Zellen lieferten hohe Mengen an aktiven CAR-Fusionsproteinen im Überstand und wurden daher bevorzugt eingesetzt. COS-1 Zellen wurden bei einer Konfluenz von 80-90% mit Ad4679 bzw. Ad5091 mit einer MOI von 10-50 in DMEM (mit 2 % fötalem Kälberserum supplementiert) infiziert und 48 h bei 37°C und 5% CO₂ inkubiert. Nach 48 h wurden die infizierten Zellkulturen mit 1/10 Vol. 10x Auftragspuffer (500 mM NaH₂PO₄, 3 M NaCl, 100 mM Imidazol) versetzt und 1 min weiter im Brutschrank inkubiert. Danach wurde der Zellkulturüberstand für die nachfolgende säulenchromatographische Aufreinigung abgenommen (die infizierten Zellen wurden für eine weitere Inkubationsperiode mit Medium versetzt und wieder 48 h inkubiert). Die gewonnenen Überstände wurden gesammelt, eventuell verschleppte Zellen durch eine 10minütige Zentrifugation bei 1000 x g und eine Sterilfiltration mit einem 0,22 µm Filter abgetrennt. Danach wurde die Säulenchromatographie durchgeführt. Hierzu wurde Ni-NTA-Agarose (Qiagen) mit Auftragspuffer (50 mM NaH₂PO₄, 300 mM NaCl, 10 mM Imidazol) äquilibriert und die oben beschriebenen Zellkulturüberstände aufgetragen. Anschließend wurde die Säule mit Waschpuffer (50 mM NaH₂PO₄, 300 mM NaCl, 20 mM Imidazol) gewaschen.

Die Elution der CAR-Fusionsproteine erfolgte mit Elutionspuffer (50 mM NaH₂PO₄, 300 mM NaCl, 50 mM L-Histidin). Die Konzentration und Funktion der CAR-Fusionsproteine im Eluat wurde durch BioRad Protein Assay, SDS-Gelelektrophorese mit Sypro-Orange Färbung sowie Infektionsexperimente ermittelt. Ausreichend konzentrierte Eluate wurden gegen 25 % Glycerol in DMEM-Medium dialysiert, in flüssigem Stickstoff schockgefroren und bei 80°C gelagert.

## Patentansprüche

1. Adapter zum Ankoppeln einer an einer Zelloberfläche anzukoppelnden Substanz, umfassend:
a) einen Abschnitt zum Erkennen der und Binden an die anzukoppelnde Substanz, und
b) einen rekombinanten Abschnitt zum Anordnen des Adapters an der Zelloberfläche mit einer Affinität für eine oder mehrere negativ geladene Zelloberflächenstrukturen.

2. Adapter nach Anspruch 1, umfassend einen Abschnitt zum Erkennen des und Binden an das adenovirale Fiberknob-Protein als Abschnitt a), wobei der Abschnitt einen Teil der extrazellulären Domäne des Coxsackie Adenovirus Rezeptors (CAR) oder einen funktionsgleichen Teil umfasst.

3. Adapter nach einem der Ansprüche 1 oder 2, **dadurch gekennzeichnet, dass** der Abschnitt b) des Adapters ausgewählt ist aus:
- einem Peptid von 6 oder mehr Aminosäuren Länge, von denen zumindest die Hälfte Guanidino- und/oder Amidinoseitenketten enthalten,
- einem Oligoargininpeptid mit zumindest 6 Argininseitenketten,
- einem Peptid der Aminosäuresequenz entsprechend der basischen Region des HIV tat-Proteins,
- der dritten Helix eines Homöobox-Proteins, und
- einen die Verankerung an einer Zelloberfläche vermittelnden Abschnitt des Protein VP22 des Herpes simplex-Virus.

4. Adapter nach einem der vorherigen Ansprüche, ferner umfassend einen Oligomerisierungsabschnitt zum Bilden von Di-, Tri- und/oder Oligomeren des Adapters.

5. Adapter nach einem der vorherigen Ansprüche, ferner umfassend eine Leadersequenz, um die Synthese des Adapters in das rauhe endoplasmatische Retikulum zu bewirken.

6. Nucleinsäure mit einem Abschnitt, der für einen Adapter nach einem der vorherigen Ansprüche codiert.

7. Virus zum Exprimieren eines Adapters nach einem der Ansprüche 1 bis 5, wobei das Virus in einem Stadium seiner Vermehrung eine Virus-Hülle besitzt, und wobei das Virus enthält:
- eine Nucleinsäure gemäß Anspruch 6, und
- einen Nucleinsäure-Abschnitt zur Expression eines Proteins der Virus-Hülle, an das der Abschnitt a) des Adapters ankoppeln kann.

8. Verwendung eines Adapters nach einem der Ansprüche 1 bis 5 zum nicht-therapeutischen Vermitteln und/oder Verbessern des Ankoppelns einer Substanz an eine Zelloberfläche.

9. Verfahren zum nicht-therapeutischen Ankoppeln einer Substanz an eine Zelloberfläche, umfassend die Schritte:
a) Exponieren der anzukoppelnden Substanz gegen einen Adapter nach einem der Ansprüche 1 bis 5, um die Substanz mit dem Adapter zu versehen, und
b) Exponieren der Zelloberfläche gegen die mit dem Adapter versehene, anzukoppelnde Substanz.

10. Verwendung eines Adapters nach einem der Ansprüche 1 bis 5 zum Herstellen eines Arzneimittels zum Vermitteln und/oder Verbessern des Ankoppelns einer Substanz an eine Zelloberfläche.

11. Verwendung eines Virus nach Anspruch 7 zum Herstellen eines Arzneimittels zum Vermitteln und/oder Verbessern des Ankoppelns einer Substanz an eine Zelloberfläche.

12. Arzneimittel zum Vermitteln und/oder Verbessern des Ankoppelns einer Substanz an eine Zelloberfläche, umfassend
- einen Adapter nach einem der Ansprüche 1 bis 5 und/oder ein Virus nach Anspruch 7, und
- einen pharmazeutisch akzeptablen Träger.
